# EUROPEAN PATENT SPECIFICATION

(11) **EP 4 035 721 B1**
(45) Date of publication and mention of the grant of the patent: **20.08.2025**
(21) Application number: 20873202.4
(22) Date of filing: 29.09.2020
(51) Int. Cl.: A61M 29/02, A61M 25/00, A61B 18/14, A61B 17/02

(54) **MEDICAL DEVICE**
MEDIZINISCHE VORRICHTUNG
DISPOSITIF MÉDICAL

(30) Priority: 30.09.2019 JP 2019179477
(43) Date of publication of application: 03.08.2022
(73) Proprietor: TERUMO Kabushiki Kaisha, Tokyo 151-0072 (JP)
(72) Inventor: TAKAHASHI, Yusuke, Ashigarakami-gun, Kanagawa 259-0151 (JP)
(74) Representative: Casalonga
(86) International application number: PCT/JP2020/036815
(87) International publication number: WO 2021/065873

(56) References cited:
- WO-A1-2015/186626
- WO-A1-2018/016529
- WO-A1-2019/009254
- WO-A1-2019/181634
- JP-A- 2005 270 394
- US-A1- 2003 023 260
- US-A1- 2007 083 194
- US-A1- 2010 312 338
- US-A1- 2013 172 883
- US-A1- 2019 167 950
- US-B1- 6 423 058

## Description

### Technical Field

The present invention relates to a medical device that expands and maintains a hole of a biological tissue. In particular, the present invention relates to a medical device according to the preamble of claim 1, such as it is e.g. known from US2019/167950 A1, US 2007/083194 A1 or US2003/023260 A1.

### Background Art

Chronic heart failure is one of known heart diseases. The chronic heart failure is broadly classified into a systolic heart failure and a diastolic heart failure, based on a cardiac function index. In a patient suffering from the diastolic heart failure, a myocardium is hypertrophied and increases in stiffness (hardness), so that the blood pressure in a left atrium increases and the pumping function of a heart is decreased. Accordingly, the patient shows a heart failure symptom such as a pulmonary edema. There is also a heart disease in which the blood pressure on a right atrium side increases due to pulmonary hypertension or the like, and the pump function of a heart is decreased, thereby showing heart failure symptoms.

In recent years, for the patients suffering from a heart failure, attention has been paid to a shunt treatment in which a shunt (through-hole) serving as an escape route for increased atrial pressure is formed in an atrial septum, thereby being able to reduce heart failure symptoms. In the shunt treatment, the atrial septum is accessed using a transvenous approach method, and a through-hole is formed. Then, a method has been known in which a through-hole is widened to a desired size and the through-hole is subjected to energy and cauterized, to form a shunt hole.

In addition, a method for widening a formed hole in a biological lumen is performed in cases other than the case of forming a shunt hole in the atrial septum. For example, PTL 1 discloses a device that cuts and widens a blood vessel that is narrowed by arterioscleosis. In the device, an outer shaft is fixed to proximal portions of a plurality of expandable portions extending in an axial direction at a distal portion of the device, and an inner shaft penetrating through the outer shaft is fixed to distal portions of the plurality of expandable portions. Therefore, when the inner shaft is pulled to a proximal side with respect to the outer shaft, a compression force acts on the expandable portions, and the expandable portions are expanded to bend outward in a radial direction.

### Citation List

### Patent Literature

PTL 1: JP-A-2018-23840

### Summary of Invention

### Technical Problem

In the device disclosed in PTL 1, when the inner shaft is pulled to expand the expandable portions, the inner shaft may be bent, and a central axis of the expandable portions and a pulling axis of the inner shaft may be displaced from each other. In this case, the plurality of expandable portions arranged in a circumferential direction are not uniform, and an expansion force of the expandable portions is not uniform. Accordingly, the expansion force may be decreased, or a hole in a living body may be not expandable to a desired shape.

On the other hand, when a tube side shaft to be pulled so as to expand the expandable portions is made rigid, since the device is difficult to bend, the passability of the device in a delivery sheath that allows the device to reach a target site, or in a biological lumen such as a blood vessel is decreased.

The invention is conceived in view of the aforementioned problems, and an object of the invention is to provide a medical device capable of improving passability in a tubular member or in a biological lumen, and suppressing a decrease in expansion force to widen a biological tissue.

### Solution to Problem

In order to solve the above-mentioned problem, the present invention provides a medical device according to independent claim 1. The dependent claims relate to advantageous embodiments.

### Advantageous Effect of Invention

In the medical device configured as described above, in the contracted form where the inner tube is extracted from the outer tube, a range where the outer tube and the inner tube overlap each other between the first connecting portion and the second connecting portion is shortened. For this reason, in the contracted form where the expansion body is contracted, the flexibility of the medical device between the first connecting portion and the second connecting portion is improved, and the passability of the medical device in a tubular member such as a sheath or in a biological lumen is improved. In addition, in the medical device, in the reference form where the expansion body is expanded, the range where the outer tube and the inner tube overlap each other between the first connecting portion and the second connecting portion is lengthened. For this reason, in the medical device, the shaft portion is difficult to bend between the first connecting portion and the second connecting portion. For this reason, the medical device can maintain the expansion body in a proper shape in the reference form, so that a decrease in expansion force can be suppressed.

The expansion body may be deformable to be in an expanded form, where the expansion body is expanded in the radial direction, from the reference form by the first connecting portion and the second connecting portion approach each other, and when the expansion body is deformed from the reference form into the expanded form, a part of the inner tube may be stored inside the outer tube from the opening portion. Accordingly, even when a compression force is acted on the expansion body in the axial direction to set the expansion body to the expanded form where the expansion body is more expanded in the radial direction than in the reference form, the shaft portion is difficult to bend between the first connecting portion and the second connecting portion, so that buckling can be suppressed. For this reason, in the medical device, the expansion body is deformable to be in the expanded form of a desired shape that is uniform in a circumferential direction, so that a decrease in expansion force can be suppressed and a biological tissue can be uniformly widened in the radial direction.

The outer tube and/or the inner tube may include a flexible portion having lower flexural rigidity than a portion adjacent to the flexible portion in the axial direction. In the reference form, the flexible portion may be located in a range where the outer tube and the inner tube overlap each other. In the contracted form, the flexible portion may be located in a range different from the range where the outer tube and the inner tube overlap each other. Accordingly, in the contracted form, the flexible portion is located outside the range where the outer tube and the inner tube overlap each other, so that the flexible portion can be flexibly bent. For this reason, in the contracted form, the flexibility of the medical device between the first connecting portion and the second connecting portion is improved, and the passability of the medical device in a tubular member such as a sheath or in a biological lumen is improved. In addition, in the reference form, since the flexible portion is located in the range where the outer tube and the inner tube overlap each other, the medical device is difficult to bend between the first connecting portion and the second connecting portion. Therefore, the medical device can maintain the expansion body in a proper shape in the reference form, so that a decrease in expansion force can be suppressed and a biological tissue can be uniformly widened in the radial direction.

The outer tube may include a first engagement portion. The inner tube may include a second engagement portion. At least in the reference form, the first engagement portion and the second engagement portion may be slidable in the axial direction, and come into contact with each other in a circumferential direction to limit relative rotation between the outer tube and the inner tube. Accordingly, at least in the reference form, relative rotation between the outer tube and the inner tube is limited. For this reason, at least in the reference form, the twisting of the expansion body can be suppressed. Therefore, in the medical device, the expansion body is deformable to be in the reference form of a desired shape, and a decrease in expansion force can be suppressed.

A flexible portion may be formed of a slit portion having a spiral shape or a groove provided in the outer tube and/or in the inner tube. Accordingly, the flexible portion of the outer tube and/or the inner tube can be flexibly bent and easily processed.

A flexible portion may be formed of a plurality of wires. Accordingly, the flexible portion of the outer tube and/or the inner tube can be flexibly bent.

A flexible portion may be formed in a coil shape. Accordingly, the flexible portion of the outer tube and/or the inner tube can be flexibly bent.

A flexible portion may be made of a material softer than a material of a portion adjacent to the flexible portion in the axial direction. Accordingly, the flexible portion of the outer tube and/or the inner tube can be flexibly bent.

### Brief Description of Drawings

[Fig. 1] Fig. 1 is a front view illustrating an overall configuration of a medical device according to the present embodiment.
[Fig. 2] Fig. 2 is an enlarged perspective view of the vicinity of an expansion body.
[Fig. 3] Fig. 3 illustrates front views of the vicinity of an outer tube and an inner tube through a storage sheath and through the expansion body, Fig. 3(A) illustrates a contracted form where the expansion body is contracted, Fig. 3(B) illustrates a reference form where the expansion body is in an original shape, and Fig. 3(C) illustrates an expanded form where the expansion body is expanded.
[Fig. 4] Fig. 4 is a view for describing a treatment method using the medical device according to the present embodiment, and is a view for schematically describing a state where the expansion body is disposed in a through-hole of an atrial septum, in which the medical device and a biological tissue are illustrated in a front view and in a cross-sectional view, respectively.
[Fig. 5] Fig. 5 is a front view illustrating the medical device that is bent in a delivery sheath, through the storage sheath and through the expansion body.
[Fig. 6] Fig. 6 is a view for schematically describing a state where the expansion body is disposed in the atrial septum, in which the medical device and the biological tissue are illustrated in a front view and in a cross-sectional view, respectively.
[Fig. 7] Fig. 7 is a view for schematically describing a state where the expansion body is expanded in diameter in the atrial septum, in which the medical device and the biological tissue are illustrated in a front view and in a cross-sectional view, respectively.
[Fig. 8] Fig. 8 illustrates plan views of the vicinity of an outer tube and an inner tube of a medical device according to a first modification example through a storage sheath and through an expansion body, Fig. 8(A) illustrates a contracted form, and Fig. 8(B) illustrates a reference form.
[Fig. 9] Fig. 9 illustrates plan views of the vicinity of an outer tube and an inner tube of a medical device according to a second modification example through a storage sheath and through an expansion body, Fig. 9(A) illustrates a contracted form, and Fig. 9(B) illustrates a reference form.
[Fig. 10] Fig. 10 illustrates plan views of the vicinity of an outer tube and an inner tube of a medical device according to a further modification example (not falling under the scope of the claims), Fig. 10(A) illustrates an extended form, and Fig. 10(B) illustrates an accommodated form.
[Fig. 11] Fig. 11 illustrates plan views of the vicinities of flexible portions of modification examples in a medical device, Fig. 11(A) illustrates a fourth modification example, Fig. 11(B) illustrates a fifth modification example, Fig. 11(C) illustrates a sixth modification example, Fig. 11(D) illustrates a seventh modification example, and Fig. 11(E) illustrates an eighth modification example.
[Fig. 12] Fig. 12 is a front view of an expanded form of an embodiment not falling under the scope of the invention illustrating the vicinity of the outer tube and the inner tube through the storage sheath and through the expansion body.

### Description of Embodiments

Hereinafter, embodiments of the invention will be described with reference to the accompanying drawings. Note that dimensional ratios in the drawings may be exaggerated and different from actual ratios for convenience of description. In addition, in the specification, a side on which a medical device 10 is inserted into a biological lumen will be referred to as a "distal side", and a side on which operation is performed will be referred to as a "proximal side".

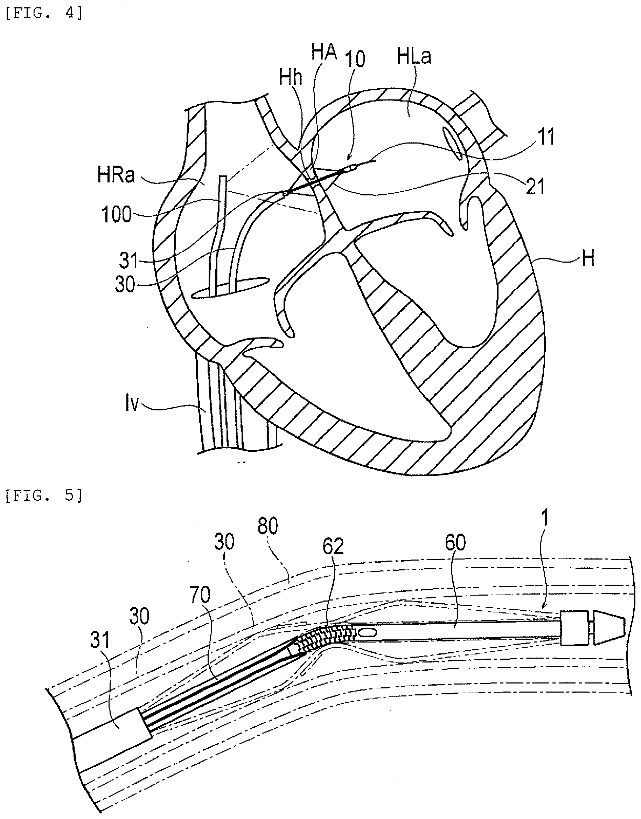

As illustrated in Fig. 4, the medical device 10 according to the present embodiment is configured to be able to expand a through-hole Hh formed in an atrial septum HA of a heart H of a patient and to perform a maintenance treatment to maintain the size of the expanded through-hole Hh.

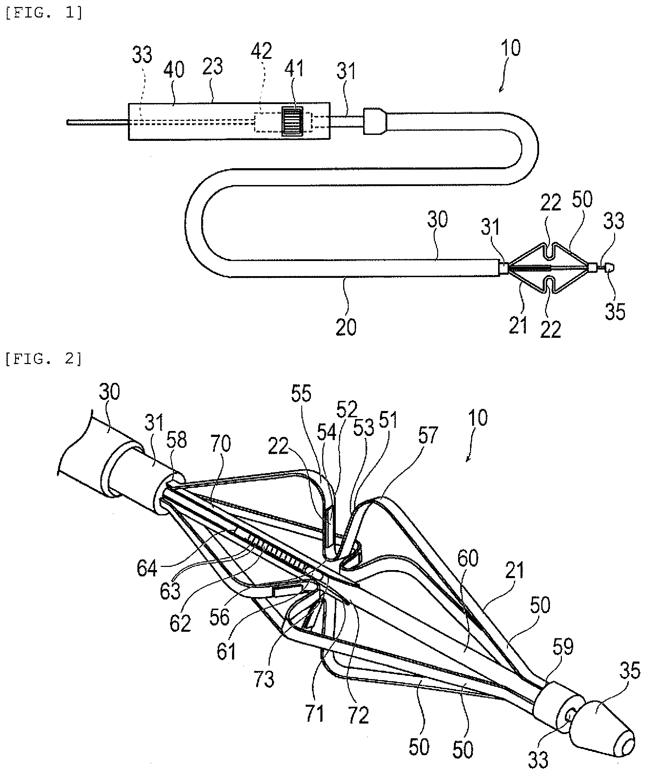

As illustrated in Figs. 1 and 2, the medical device 10 of the present embodiment includes a shaft portion 20 that is elongate, an expansion body 21 provided at a distal portion of the shaft portion 20, a pulling shaft 33 that expands the expansion body 21, and an operation unit 23 provided at a proximal portion of the shaft portion 20. The expansion body 21 is provided with an energy transmission element 22 for performing the aforementioned maintenance treatment.

The shaft portion 20 includes a main shaft 31 that holds the expansion body 21 at a distal portion of the main shaft 31, a storage sheath 30 that stores the main shaft 31, an outer tube 70 connected to the distal portion of the main shaft 31, and an inner tube 60 that can be stored in the outer tube 70. The storage sheath 30 is movable forward and backward with respect to the main shaft 31 in an axial direction. In a state where the storage sheath 30 is moved to a distal side of the shaft portion 20, the storage sheath 30 can store the expansion body 21 thereinside. The storage sheath 30 is moved to the proximal side from a state where the expansion body 21 is stored, and thus the expansion body 21 can be exposed.

A proximal portion of the main shaft 31 is connected to the operation unit 23. The distal portion of the main shaft 31 is connected to a proximal portion of the expansion body 21 and to a proximal portion of the outer tube 70. The outer tube 70 extends to the distal side from the distal portion of the main shaft 31.

The pulling shaft 33 is stored inside the main shaft 31, the outer tube 70, and the inner tube 60. The pulling shaft 33 is a shaft for pulling to act a compression force on the expansion body 21. An axially orthogonal cross section of an outer peripheral surface of the pulling shaft 33 is a substantially circular shape. The pulling shaft 33 protrudes from a distal end of the inner tube 60 to the distal side, and a distal portion of the pulling shaft 33 is connected to a distal member 35. A proximal portion of the pulling shaft 33 is led out to the proximal side from the operation unit 23. The distal member 35 to which the distal portion of the pulling shaft 33 is fixed may not be fixed to the expansion body 21. Accordingly, the distal member 35 can pull the expansion body 21 in a compression direction. In addition, when the expansion body 21 is stored in the storage sheath 30, the distal member 35 is separated to the distal side from the expansion body 21, so that the expansion body 21 can be easily moved in a stretching direction and storability can be improved.

The operation unit 23 includes a housing 40 to be gripped by an operator, an operation dial 41 to be rotationally operable by the operator, and a conversion mechanism 42 that operates in conjunction with rotation of the operation dial 41. The pulling shaft 33 is held by the conversion mechanism 42 inside the operation unit 23. The conversion mechanism 42 can move the held pulling shaft 33 forward and backward along the axial direction with rotation of the operation dial 41. For example, a rack and pinion mechanism can be used as the conversion mechanism 42.

As illustrated in Fig. 2, the expansion body 21 includes a plurality of wire portions 50 in a circumferential direction. In the present embodiment, four wire portions 50 are provided in the circumferential direction. Note that the number of the wire portions 50 is not particularly limited. Each of the wire portions 50 is expandable and contractable in a radial direction of the expansion body 21. A proximal portion of the wire portion 50 is connected to a first connecting portion 58 provided at the distal portion of the main shaft 31. The first connecting portion 58 located at the proximal portion of the wire portion 50 is connected to the proximal portion of the outer tube 60 and to the distal portion of the main shaft 31. A second connecting portion 59 located at a distal portion of the wire portion 50 is connected to a distal portion of the inner tube 60. The distal portion of the wire portion 50 extends from the distal portion of the inner tube 60 to the proximal side. The wire portion 50 is inclined such that the size in the radial direction increases from both end portions toward a central portion in the axial direction. In addition, the wire portion 50 includes a holding portion 51 having a valley shape in the radial direction of the expansion body 21, at the central portion of the wire portion 50 in the axial direction.

The holding portion 51 includes a proximal side holding portion 52, and a distal side holding portion 53 located closer to the distal side than the proximal side holding portion 52. The holding portion 51 further includes a proximal side outward projection portion 55, an inward projection portion 56, and a distal side outward projection portion 57. It is preferable that an interval between the proximal side holding portion 52 and the distal side holding portion 53 in the axial direction is slightly wider on a radially outward side than on a radially inward side, in a natural state where no external force acts thereon. Accordingly, a biological tissue is easily disposed between the proximal side holding portion 52 and the distal side holding portion 53 from the radially outward side.

The proximal side holding portion 52 includes a projection portion 54 protruding toward the distal side. The energy transmission element 22 is disposed on the projection portion 54. Note that the proximal side holding portion 52 may not include the projection portion 54. Namely, the energy transmission element 22 may not protrude to the distal side.

The proximal side outward projection portion 55 is located on a proximal side of the proximal side holding portion 52, and is formed in a shape projecting outward in the radial direction. The distal side outward projection portion 57 is located on a distal side of the distal side holding portion 53, and is formed in a shape projecting outward in the radial direction. The inward projection portion 56 is located between the proximal side holding portion 52 and the distal side holding portion 53, and is formed in a shape projecting inward in the radial direction. The proximal side outward projection portion 55, the inward projection portion 56, and the distal side outward projection portion 57 are stored in the storage sheath 30, thus being deformable from a projection shape into a shape close to being flat.

In the present embodiment, the energy transmission element 22 is provided at the proximal side holding portion 52, but the energy transmission element 22 may be provided at the distal side holding portion 53.

Each of the wire portions 50 forming the expansion body 21 has, for example, a flat plate shape obtained by cutting a cylinder. A wire forming the expansion body 21 can have a thickness of 50 to 500 µm and a width of 0.3 to 2.0 mm. However, a wire forming the expansion body 21 may have dimensions outside these ranges. In addition, the shape of the wire portion 50 is not limited, and may have, for example, a circular cross-sectional shape or other cross-sectional shapes.

Since the energy transmission element 22 is provided at the projection portion 54 of the proximal side holding portion 52, when the holding portion 51 holds the atrial septum HA, energy from the energy transmission element 22 is transmitted from a right atrium side to the atrial septum HA. Note that when the energy transmission element 22 is provided at the distal side holding portion 53, energy from the energy transmission element 22 is transmitted from a left atrium side to the atrial septum HA.

The energy transmission element 22 is configured as, for example, a bipolar electrode that receives electric energy from an energy supply device (not illustrated) that is an external device. In this case, energization is performed between the energy transmission elements 22 disposed on the wire portions 50. The energy transmission element 22 and the energy supply device are connected to each other by a conducting wire (not illustrated) coated with an insulating coating material. The conducting wire is led out to the outside via the shaft portion 20 and via the operation unit 23, and is connected to the energy supply device.

Alternatively, the energy transmission element 22 may be configured as a monopolar electrode. In this case, energization is performed between the energy transmission element 22 and a counter electrode plate prepared outside a body. In addition, the energy transmission element 22 may be a heating element (electrode chip) that receives high-frequency electric energy from the energy supply device to generate heat. In this case, energization is performed between the energy transmission elements 22 disposed on the wire portions 50. Further, the energy transmission element 22 can be configured as an element capable of applying energy to the through-hole Hh, such as an element that exerts a heating or cooling action using microwave energy, ultrasound energy, coherent light such as laser, a heated fluid, a cooled fluid, or a chemical medium, an element that generates frictional heat, or a heater including an electric wire or the like, and a specific form of the energy transmission element 22 is not particularly limited.

The wire portion 50 can be made of a metallic material. As the metallic material, for example, a titanium-based (Ti-Ni, Ti-Pd, Ti-Nb-Sn, or the like) alloy, a copper-based alloy, stainless steel, β-titanium steel, or a Co-Cr alloy can be used. Note that it is better to use an alloy or the like having a spring property such as a nickel-titanium alloy. However, the material for the wire portion 50 is not limited to these materials, and the wire portion 50 may be made of other materials.

The pulling shaft 33 is stored inside the shaft portion 20. A guide wire lumen is formed in the pulling shaft 33 and in the distal member 35 along the axial direction, and a guide wire 11 can be inserted into the guide wire lumen.

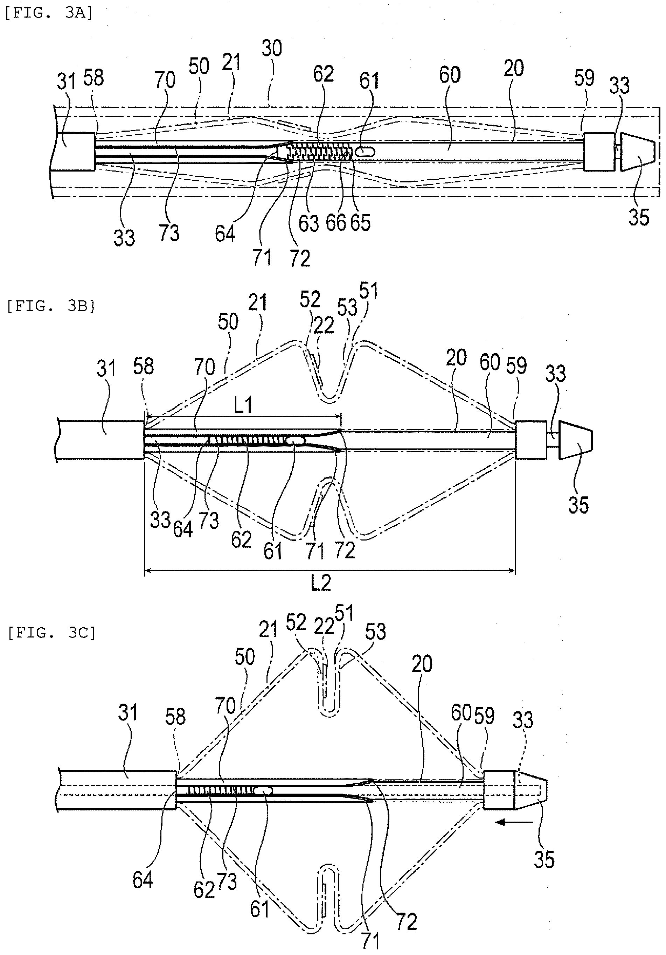

Next, the outer tube 70 and the inner tube 60 will be described. As illustrated in Figs. 2 and 3(B), the outer tube 70 extends to the distal side from the first connecting portion 58 at the proximal portion of the expansion body 21. The outer tube 70 includes an open end 72 at which an opening portion 71 is formed, on the distal side. In addition, a first engagement portion 73 having a slit shape and extending from the open end 72 to the proximal side along the axial direction is formed in the outer tube 70. The first engagement portion 73 has such a width in the circumferential direction that a second engagement portion 61 formed in the inner tube 60 can enter the first engagement portion 73. It is preferable that the width of the first engagement portion 73 in the circumferential direction is widened at a distal portion of the first engagement portion 73. Accordingly, the first engagement portion 73 easily receives the second engagement portion 61 from the distal side. The outer tube 70 includes one first engagement portion 73, but may include two or more first engagement portions 73 at different locations in the circumferential direction. In a natural state where no external force acts on the expansion body 21, the expansion body 21 is in a reference form where the expansion body 21 is deployed in the radial direction. In the reference form, the open end 72 is located closer to the distal side than the first connecting portion 58, and is closer to the proximal side than the second connecting portion 59.

In the reference form where the expansion body 21 is widened with no external force acting on the expansion body 21, a distance L1 from the first connecting portion 58 to the open end 72 is 30% to 80% of a distance L2 from the first connecting portion 58 to the second connecting portion 59. When the distance L1 is too short, the range where the outer tube 70 and the inner tube 60 overlap each other between the first connecting portion 58 and the second connecting portion 59 is shortened, so that the effect of making the shaft portion difficult to bend in the reference form is decreased. When the distance L1 is too long, the range where the outer tube 70 and the inner tube 60 overlap each other between the first connecting portion 58 and the second connecting portion 59 is lengthened, so that the effect of making the shaft portion 20 easy to bend in a contracted form where the expansion body 21 is contracted (refer to Fig. 3(A)) is decreased.

The inner tube 60 is slidable in the axial direction inside the outer tube 70. The inner tube 60 extends to the proximal side from the second connecting portion 59 at the distal portion of the expansion body 21. A proximalmost end 64 of the inner tube 60 is located closer to the proximal side than the open end 72 of the outer tube 70. The inner tube 60 includes a flexible portion 62 that has lower flexural rigidity and is easier to bend than a portion adjacent to the flexible portion 62 in the axial direction. The flexible portion 62 can be disposed inside the outer tube 70 as illustrated in Figs. 3(B) and 3(C), and can be extracted from the outer tube 70 to the distal side and disposed closer to the distal side than the outer tube 70 as illustrated in Fig. 3(A). The flexible portion 62 is formed in a spiral shape by forming a slit portion 63 having a spiral shape and penetrating through the flexible portion 62 from an outer peripheral surface to an inner peripheral surface. The slit portion 63 can be easily formed by, for example, laser processing. Protruding portions 66 to be fitted to a recessed portion 65 and to a recessed portion 65 are formed in surfaces that face each other while interposing the slit portion 63 therebetween. The protruding portions 66 are widened on a protruding direction side. For this reason, the protruding portion 66 has a structure where the protruding portion 66 does not come off from the recessed portion 65. Therefore, the flexible portion 62 has a structure where the flexible portion 62 is easy to bend but is strong against a tensile force. In addition, the second engagement portion 61 protruding outward in the radial direction is formed on an outer peripheral surface of the inner tube 60. As illustrated in Figs. 3(B) and 3(C), the second engagement portion 61 can slidably enter the first engagement portion 73 of the outer tube 70. The second engagement portion 61 is formed closer to the distal side than the flexible portion 62, but the position of the second engagement portion 61 is not particularly limited. Therefore, the second engagement portion 61 may be formed closer to the proximal side than the flexible portion 62, or may be formed to overlap the flexible portion 62.

It is preferable that the storage sheath 30 and the main shaft 31 of the shaft portion 20 are made of a material having a certain degree of flexibility. Examples of such a material include polyolefins such as polyethylene, polypropylene, polybutene, ethylene-propylene copolymer, ethylene-vinyl acetate copolymer, ionomer, and a mixture of two or more thereof, fluororesins such as soft polyvinyl chloride resin, polyamide, polyamide elastomer, polyether blockamide, polyester, polyester elastomer, polyurethane, and polytetrafluoroethylene, polyimide, PEEK, silicone rubber, and latex rubber.

The pulling shaft 33 can be made of, for example, an elongate wire such as a metallic material such as stainless steel or a super-elastic alloy such as a nickel-titanium alloy or a copper-zinc alloy, or a resin material having relatively high rigidity. In addition, the pulling shaft 33 may be formed by coating the above material with a resin material such as polyvinyl chloride, polyethylene, polypropylene, ethylene-propylene copolymer, or fluororesin.

The distal member 35, the inner tube 60, and the outer tube 70 can be made of, for example, a metallic material such as stainless steel or a super-elastic alloy such as a nickel-titanium alloy or a copper-zinc alloy, or a resin material having relatively high rigidity.

Next, a treatment method using the medical device 10 according to the present embodiment will be described. The treatment method is performed on a patient suffering from a heart failure (left heart failure). More specifically, as illustrated in Fig. 4, the treatment method is performed on a patient suffering from a chronic heart failure in which a myocardium of a left ventricle of the heart H is hypertrophied and increases in stiffness (hardness) to cause an increase in blood pressure in a left atrium HLa.

When the operator forms the through-hole Hh, the operator delivers an introducer in which a guiding sheath and a dilator are combined together, to the vicinity of the atrial septum HA. The introducer can be delivered to, for example, a right atrium HRa via an inferior vena cava Iv. In addition, the introducer can be delivered using the guide wire 11. The operator can insert the guide wire 11 into the dilator, and deliver the introducer along the guide wire 11. Note that the insertion of the introducer into or the insertion of the guide wire 11 into a living body can be performed using a known method such as using an introducer for blood vessel introduction.

Next, the operator causes a puncture device (not illustrated) and the dilator to penetrate through the atrial septum HA from a right atrium HRa side toward a left atrium HLa side to form the through-hole Hh. For example, a device such as a wire having a sharp distal end can be used as the puncture device. The puncture device is inserted into the dilator, and is delivered to the atrial septum HA. After the guide wire 11 is removed from the dilator, instead of the guide wire 11, the puncture device can be delivered to the atrial septum HA.

Next, the operator delivers the medical device 10 to the vicinity of the atrial septum HA along the guide wire 11 inserted into the left atrium HLa from the right atrium HRa via the through-hole Hh in advance. At this time, a part of a distal portion of the medical device 10 passes through the through-hole Hh opened in the atrial septum HA, and reaches the left atrium HLa. In addition, when the medical device 10 is inserted, as illustrated in Fig. 3(A), the expansion body 21 is in the contracted form where the expansion body 21 is stored in the storage sheath 30. In the contracted form, the proximal side outward projection portion 55, the inward projection portion 56, and the distal side outward projection portion 57 that have a projection shape in the reference form are elastically deformed into a shape close to being flat, so that the expansion body 21 is contracted in the radial direction. In the contracted form, the flexible portion 62 of the inner tube 60 is located closer to the distal side than the outer tube 70. For this reason, the flexible portion 62 is not covered with the outer tube 70. Accordingly, the flexible portion 62 can be flexibly bent. Therefore, as illustrated in Fig. 5, when the flexible portion 62 is moved in a delivery sheath 80 for transporting the distal portion of the medical device 10 to a target position in the living body, or in a blood vessel, the flexible portion 62 can be easily bent according to the bending of the delivery sheath 80 or of the blood vessel. For this reason, the passability of the medical device 10 in the delivery sheath 80 or in the blood vessel is improved.

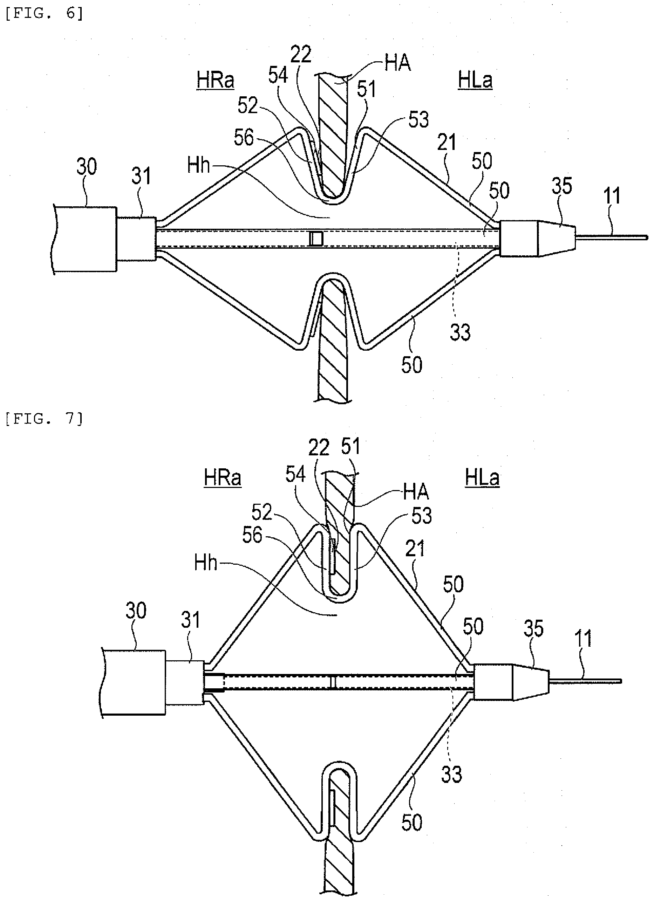

Next, the storage sheath 30 is moved to the proximal side to expose a distal side portion of the expansion body 21 into the left atrium HLa. Accordingly, the distal side portion of the expansion body 21 is deployed in the radial direction inside the left atrium HLa by its own restoring force. Next, as illustrated in Figs. 3(B) and 6, the storage sheath 30 is moved to the proximal side to expose the entirety of the expansion body 21. Accordingly, a proximal side portion of the expansion body 21 is deployed in the radial direction inside the right atrium HRa by its own restoring force. At this time, the inward projection portion 56 is disposed inside the through-hole Hh. Accordingly, the entirety of the expansion body 21 is deployed by its own elastic force, and restores to the original reference form or to a form close to the reference form. At this time, the atrial septum HA is disposed between the proximal side holding portion 52 and the distal side holding portion 53. Note that the expansion body 21 may come into contact with the through-hole Hh, thereby returning to a shape close to the reference form instead of completely returning to the reference form. Note that in this state, the expansion body 21 is not covered with the storage sheath 30 and does not receive a force from the pulling shaft 33. This form of the expansion body 21 can be defined as being included in the reference form.

When the expansion body 21 is deformed from the contracted state into the reference form, the first connecting portion 58 and the second connecting portion 59 approach each other. Accordingly, the inner tube 60 connected to the second connecting portion 59 moves to the proximal side. For this reason, the flexible portion 62 of the inner tube 60 enters the inside of the outer tube 70 from the opening portion 71. For this reason, the flexible portion 62 is surrounded by the outer tube 70, and overlaps the outer tube 70. As a result, the flexible portion 62 is difficult to bend. In addition, the range in the axial direction where the outer tube 70 and the inner tube 60 overlap each other is longer in the reference form (refer to Fig. 3(B)) and in an expanded form (refer to Fig. 3(C)) than in the contracted form (refer to Fig. 3(A)). For this reason, the outer tube 70 and the inner tube 60 between the first connecting portion 58 and the second connecting portion 59 are more difficult to bend in the reference form and in the expanded form than in the contracted form. For this reason, the expansion body 21 can be uniformly widened to cause an expansion force to uniformly act on the through-hole Hh of the atrial septum HA.

In addition, when the expansion body 21 is in the reference form, the second engagement portion 61 protruding from the outer peripheral surface of the inner tube 60 is accommodated in the first engagement portion 73 having a slit shape which is formed in the outer tube 70. Accordingly, the first engagement portion 73 and the second engagement portion 61 engage with each other, and the inner tube 60 is not rotatable with respect to the outer tube 70. For this reason, it is possible to suppress the twisting of the expansion body 21. For this reason, the expansion body 21 can be uniformly widened with a uniform expansion force to cause an expansion force to uniformly act on the through-hole Hh of the atrial septum HA.

Next, the operator operates the operation unit 23 in a state where the atrial septum HA is held by the holding portion 51, to move the pulling shaft 33 to the proximal side. Accordingly, as illustrated in Figs. 3(C) and 7, the expansion body 21 receiving a compression force in the axial direction is deformed into the expanded form where the expansion body 21 is more expanded in the radial direction than in the reference form. In the expanded form of the expansion body 21, the proximal side holding portion 52 and the distal side holding portion 53 approach each other, and the atrial septum HA is held between the proximal side holding portion 52 and the distal side holding portion 53. The holding portion 51 additionally expands in a state where the holding portion 51 holds the atrial septum HA, to widen the through-hole Hh in the radial direction.

In the expanded form, similarly to the reference form, the flexible portion 62 is surrounded by the outer tube 70, and overlaps the outer tube 70. For this reason, the flexible portion 62 is difficult to bend. In addition, since the range in the axial direction where the outer tube 70 and the inner tube 60 overlap each other is longer in the expanded form than in the contracted form, the outer tube 70 and the inner tube 60 between the first connecting portion 58 and the second connecting portion 59 are difficult to bend in the expanded form. For this reason, even when the outer tube 70 and the inner tube 60 between the first connecting portion 58 and the second connecting portion 59 receives a compression force due to pulling of the pulling shaft 33, the outer tube 70 and the inner tube 60 are difficult to buckle. Therefore, the expansion body 21 can be uniformly widened with a uniform expansion force to uniformly widen the through-hole Hh in the radial direction.

In addition, in the expanded form, similarly to the reference form, the first engagement portion 73 and the second engagement portion 61 engage with each other, and the inner tube 60 is not rotatable with respect to the outer tube 70. For this reason, it is possible to suppress the twisting of the expansion body 21. For this reason, the expansion body 21 can be uniformly widened with a uniform expansion force to uniformly widen the through-hole Hh in the radial direction.

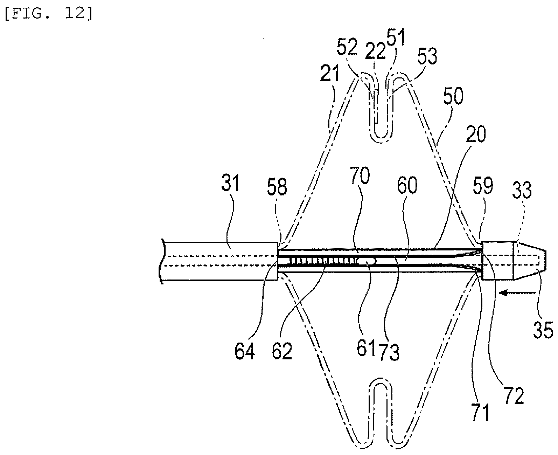

In the expanded form, when the operator moves the pulling shaft 33 further to the proximal side than in the state illustrated in Figs. 3(C) and 7, as illustrated in Fig. 12, which illustrates an embodiment not falling under the scope of the invention, the open end 72 that is a distal side end portion of the outer tube 70 abuts against the second connecting portion 59. Accordingly, a relative movement between the outer tube 70 and the inner tube 60 is limited, and excessive expansion of the expansion body 21 is limited. As a result, excessive expansion of the through-hole Hh can be limited, and safety can be improved. Note that the proximalmost end 64 of the inner tube 60 may abut against, for example, a structure protruding from an inner peripheral surface of the outer tube 70 before the open end 72 of the outer tube 70 abuts against the second connecting portion 59 when the operator moves the pulling shaft 33 to the proximal side. Even with such a configuration, a relative movement between the outer tube 70 and the inner tube 60 can be limited, and excessive expansion of the expansion body 21 can be limited. As a result, excessive expansion of the through-hole Hh can be limited, and safety can be improved.

After the through-hole Hh is expanded, hemodynamics is confirmed. As illustrated in Fig. 4, the operator delivers a hemodynamics confirmation device 100 to the right atrium HRa via the inferior vena cava Iv. For example, a known echo catheter can be used as the hemodynamics confirmation device 100. The operator can cause a display device such as a display to display an echo image acquired by the hemodynamics confirmation device 100, and confirm the amount of blood passing through the through-hole Hh, based on a display result.

Next, the operator performs a maintenance treatment to maintain the size of the through-hole Hh. In the maintenance treatment, energy is applied to an edge portion of the through-hole Hh through the energy transmission element 22, so that the edge portion of the through-hole Hh is cauterized (heated and cauterized) by the energy. When a biological tissue in the vicinity of the edge portion of the through-hole Hh is cauterized through the energy transmission element 22, a degenerated portion in which the biological tissue is degenerated is formed in the vicinity of the edge portion. Since the biological tissue in the degenerated portion is in a state where elasticity is lost, the through-hole Hh can maintain a shape when the through-hole Hh is widened by the expansion body 21.

In the expanded form, as described above, since the expansion body 21 is uniformly widened with a uniform expansion force, the energy transmission element 22 provided in each of the wire portions 50 is properly pressed against the atrial septum HA. In addition, the energy transmission element 22 is disposed on the projection portion 54 of the proximal side holding portion 52. For this reason, the maintenance treatment is performed in a state where the energy transmission element 22 is buried in the biological tissue by pressing the projection portion 54 against the atrial septum HA. Accordingly, the energy transmission element 22 does not come into contact with the blood during the maintenance treatment, so that it is possible to suppress the generation of blood clots or the like caused by the leakage of a current to the blood.

After the maintenance treatment, hemodynamics is confirmed again, and when the amount of the blood passing through the through-hole Hh reaches a desired amount, the operator reduces the expansion body 21 in diameter. The operator moves the storage sheath 30 with respect to the expansion body 21 in a distal end direction. Accordingly, the expansion body 21 is stored in the storage sheath 30 from the proximal side, and is in the contracted form. Further, the operator removes the entirety of the medical device 10 out of the living body to end the treatment.

As described above, the medical device 10 according to the aforementioned embodiment is a medical device including: the elongated shaft portion 20; and the expansion body 21 provided at the distal portion of the shaft portion 20 to be expandable and contractable in a radial direction. The shaft portion 20 includes the outer tube 70, and the inner tube 60 that is slidable in the axial direction inside the outer tube 70. The expansion body 21 includes the first connecting portion 58 connected to the outer tube 70, and the second connecting portion 59 connected to the inner tube 60. The outer tube 70 includes the open end 72 at which the opening portion 71 is formed, the inner tube 60 entering and exiting from the opening portion 71. The expansion body 21 is deformable to be in the reference form where the expansion body 21 is widened in the radial direction in a natural state, and is deformable to be in the contracted form where the expansion body 21 is contracted in the radial direction, when the first connecting portion 58 and the second connecting portion 59 are more separated from each other in the contracted form than in the reference form. In the reference form, the open end 72 is located between the first connecting portion 58 and the second connecting portion 59, and when the expansion body 21 is deformed from the reference form into the contracted form, a part of the inner tube 60 is extracted from the opening portion 71. Accordingly, in the medical device 10, in the contracted form where the inner tube 60 is extracted from the outer tube 70, the range where the outer tube 70 and the inner tube 60 overlap each other between the first connecting portion 58 and the second connecting portion 59 is shortened. For this reason, in the contracted form where the expansion body 21 is contracted, the flexibility of the medical device 10 between the first connecting portion 58 and the second connecting portion 59 is improved, and the passability of the medical device 10 in a tubular member such as the delivery sheath 80 or in a biological lumen is improved. In addition, in the medical device 10, in the reference form where the expansion body 21 is expanded, the range where the outer tube 70 and the inner tube 60 overlap each other between the first connecting portion 58 and the second connecting portion 59 is lengthened. For this reason, in the medical device 10, the shaft portion 20 is difficult to bend between the first connecting portion 58 and the second connecting portion 59. For this reason, the medical device 10 can maintain the expansion body 21 in a proper shape in the reference form, so that a decrease in expansion force can be suppressed and a biological tissue can be uniformly widened in the radial direction. Note that the first connecting portion 58 of the expansion body 21 may be directly connected to the outer tube 70 or may be indirectly connected to the outer tube 70 via another member. In addition, the second connecting portion 59 of the expansion body 21 may be directly connected to the inner tube 60 or may be indirectly connected to the inner tube 60 via another member.

In addition, the expansion body 21 is deformable to be in the expanded form, where the expansion body 21 is expanded in the radial direction, from the reference form by the first connecting portion 58 and the second connecting portion 59 approaching each other, and when the expansion body 21 is deformed from the reference form into the expanded form, a part of the inner tube 60 is stored inside the outer tube 70 from the opening portion 71. Accordingly, even when a compression force is acted on the expansion body 21 in the axial direction to set the expansion body 21 to the expanded form where the expansion body 21 is more expanded in the radial direction than in the reference form, the shaft portion 20 is difficult to bend between the first connecting portion 58 and the second connecting portion 59, so that buckling can be suppressed. For this reason, in the medical device 10, the expansion body 21 is deformable to be in the expanded form of a desired shape that is uniform in the circumferential direction, so that a decrease in expansion force can be suppressed and a biological tissue can be uniformly widened in the radial direction.

In addition, the inner tube 60 includes the flexible portion 62 having lower flexural rigidity than a portion adjacent to the flexible portion 62 in the axial direction. In the reference form, the flexible portion 62 is located in a range where the outer tube 70 and the inner tube 60 overlap each other. In the contracted form, the flexible portion 62 is located in a range different from the range where the outer tube 70 and the inner tube 60 overlap each other. Accordingly, in the contracted form, the flexible portion 62 is located outside the range where the outer tube 70 and the inner tube 60 overlap each other, so that the flexible portion 62 can be flexibly bent. For this reason, in the contracted form, the flexibility of the medical device 10 between the first connecting portion 58 and the second connecting portion 59 is improved, and the passability of the medical device 10 in a tubular member such as the delivery sheath 80 or in a biological lumen is improved. In addition, in the reference form, since the flexible portion 62 is located in the range where the outer tube 70 and the inner tube 60 overlap each other, the medical device 10 is difficult to bend between the first connecting portion 58 and the second connecting portion 59. Therefore, the medical device 10 can maintain the expansion body 21 in a proper shape in the reference form, so that a decrease in expansion force can be suppressed and a biological tissue can be uniformly widened in the radial direction.

In addition, the outer tube 70 includes the first engagement portion 73. The inner tube 60 includes the second engagement portion 61. At least in the reference form, the first engagement portion 73 and the second engagement portion 61 are slidable in the axial direction, and come into contact with each other in the circumferential direction to limit relative rotation between the outer tube 70 and the inner tube 60. Accordingly, at least in the reference form, relative rotation between the outer tube 70 and the inner tube 60 is limited. For this reason, at least in the reference form, the twisting of the expansion body 21 can be suppressed. Therefore, in the medical device 10, the expansion body 21 is deformable to be in the reference form of a desired shape, and a decrease in expansion force can be suppressed.

In addition, the flexible portion 62 is formed of the slit portion 63 having a spiral shape provided in the inner tube 60. Accordingly, the flexible portion 62 can be flexibly bent and easily processed.

The medical device according to the invention may be used in a treatment method. in which the through-hole Hh that is opened in a biological tissue is widened using the medical device 10, in which the medical device 10 includes the elongated shaft portion 20, and the expansion body 21 provided at the distal portion of the shaft portion 20 to be expandable and contractable in the radial direction, the shaft portion 20 includes the outer tube 70, and the inner tube 60 that is slidable in the axial direction inside the outer tube 70, the expansion body 21 includes the first connecting portion 58 connected to the outer tube 70, and the second connecting portion 59 connected to the inner tube 60, the outer tube 70 includes the open end 72 at which the opening portion 71 is formed, the inner tube 60 entering and exiting from the opening portion 71, and the expansion body 21 is able to be in the reference form where the expansion body 21 is widened in the radial direction, and is able to be in the contracted form where the expansion body 21 is contracted in the radial direction, when the first connecting portion 58 and the second connecting portion 59 are more separated from each other than in the reference form, the method including: transporting the expansion body 21 in a living body and inserting the expansion body 21 into the through-hole Hh that is opened in the biological tissue, with the expansion body 21 set to the contracted form; and widening the through-hole Hh using the expansion body 21 with the expansion body 21 set to the reference form inside the through-hole Hh.

In the treatment method configured as described above, in the contracted form where the inner tube 60 is extracted from the outer tube 70, the range where the outer tube 70 and the inner tube 60 overlap each other between the first connecting portion 58 and the second connecting portion 59 is shortened. For this reason, according to the treatment method, in the contracted form where the expansion body 21 is contracted, flexibility between the first connecting portion 58 and the second connecting portion 59 is improved, and passability in a tubular member such as the delivery sheath 80 or in a biological lumen is improved. In addition, in the reference form where the expansion body 21 is expanded, the range where the outer tube 70 and the inner tube 60 overlap each other between the first connecting portion 58 and the second connecting portion 59 is lengthened. For this reason, in the medical device 10, the shaft portion 20 is difficult to bend between the first connecting portion 58 and the second connecting portion 59. For this reason, according to the treatment method, the expansion body 21 can be maintained in a proper shape in the reference form, so that a decrease in expansion force can be suppressed and a biological tissue can be uniformly widened in the radial direction.

Note that the invention is not limited only to the aforementioned embodiment and various modifications can be made by those skilled in the art without departing from the technical concept of the invention. For example, the biological lumen to which the medical device 10 is applied is not limited to blood vessels, and may be, for example, a vessel, a ureter, a bile duct, an ovarian duct, a hepatic duct, a lymph duct, or the like.

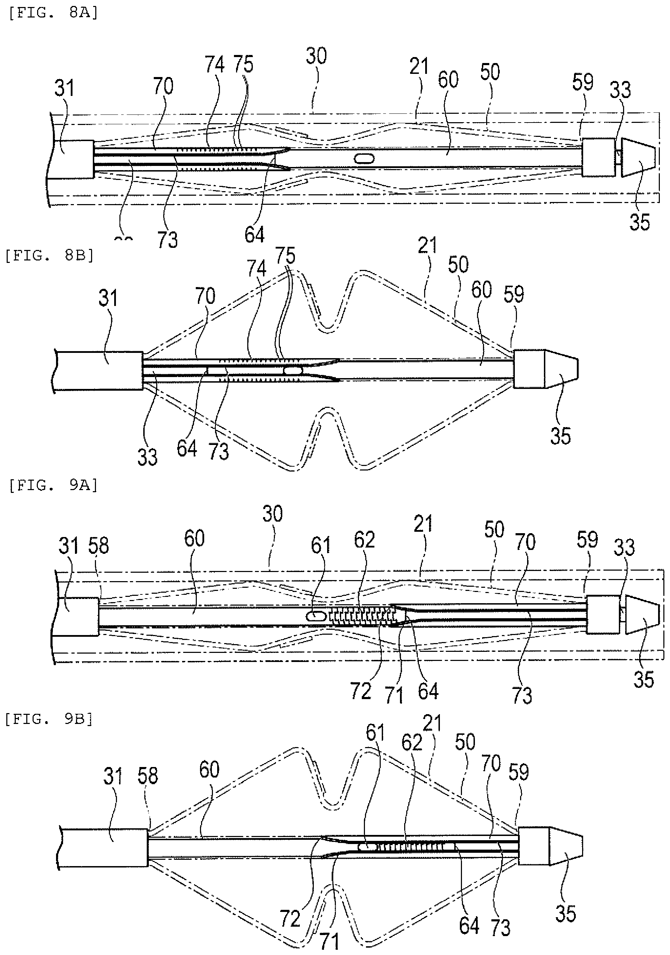

In addition, as in a first modification example illustrated in Fig. 8, a flexible portion 74 may be formed not in the inner tube 60 but in the outer tube 70. The flexible portion 74 is formed of, for example, a plurality of slit portions 75. In the contracted form, as illustrated in Fig. 8(A), the flexible portion 74 of the outer tube 70 is disposed at a position where the flexible portion 74 does not overlap the inner tube 60 in the axial direction. For this reason, the flexible portion 74 of the outer tube 70 can be easily bent. In addition, in the reference form and in the expanded form, as illustrated in Fig. 8(B), the flexible portion 74 of the outer tube 70 is disposed at a position where the flexible portion 74 overlaps the inner tube 60 in the axial direction. For this reason, the flexible portion 74 of the outer tube 70 is difficult to bend. In addition, the medical device 10 may include both the inner tube 60 including the flexible portion 74 illustrated in Fig. 3, and the outer tube 70 including the flexible portion 74 illustrated in Fig. 8.

In addition, both the inner tube 60 and the outer tube 70 may not include the flexible portion 62. The range in the axial direction where the outer tube 70 and the inner tube 60 overlap each other is longer in the reference form (refer to Fig. 3(B)) and in the expanded form (refer to Fig. 3(C)) than in the contracted form (refer to Fig. 3(A)). For this reason, even when the outer tube 70 and the inner tube 60 between the first connecting portion 58 and the second connecting portion 59 do not include the flexible portion 62, bending is more difficult in the reference form and in the expanded form than in the contracted form.

In addition, as in a second modification example illustrated in Fig. 9, the outer tube 70 may be connected to the second connecting portion 59, and the inner tube 60 may be connected to the first connecting portion 58.

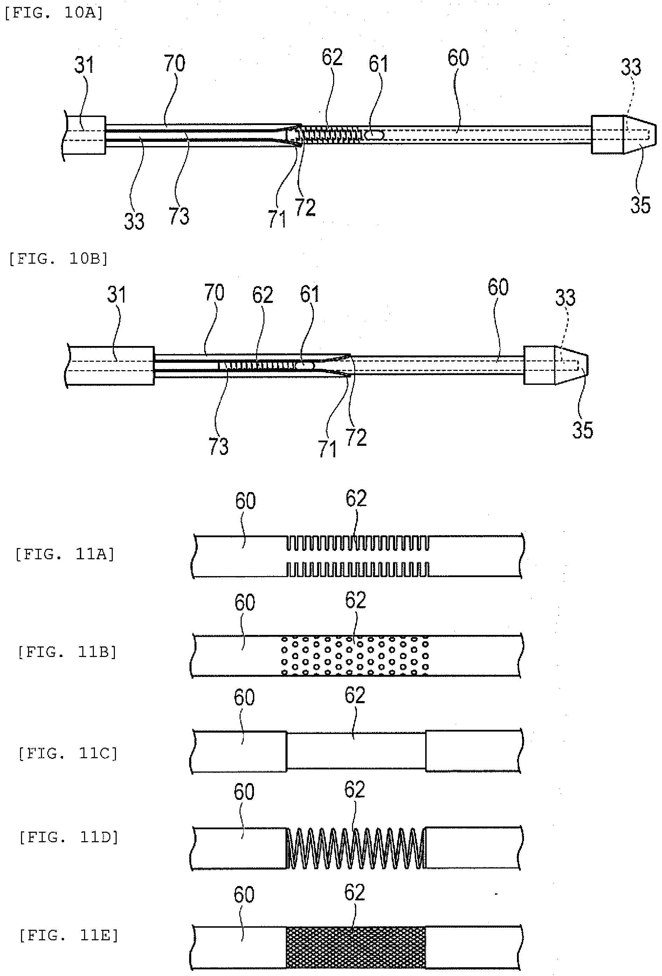

In addition, as in a third modification example illustrated in Fig. 10, the medical device 10 may not include the expansion body 21 (in which case it would not fall under the scope of the claims). The distal portion of the pulling shaft 33 is connected to the inner tube 60. When an operator pulls the pulling shaft 33, as illustrated in Fig. 10(B), the medical device 10 is set to the accommodated form where at least a part of the inner tube 60 including the flexible portion 62 is disposed at a position where at least the part overlaps the outer tube 70. In addition, when the operator pushes the pulling shaft 33, as illustrated in Fig. 10(A), the medical device 10 is set to the extended form where the flexible portion 62 of the inner tube 60 is disposed at a position where the flexible portion 62 does not overlap the outer tube 70.

As described above, the medical device 10 according to the third modification example includes the elongated shaft portion 20. The shaft portion 20 includes the outer tube 70, and the inner tube 60 that is slidable in the axial direction inside the outer tube 70. The outer tube 70 includes the open end 72 at which the opening portion 71 is formed, the inner tube 60 entering and exiting from the opening portion 71. The shaft portion 20 is able to be in the accommodated form where at least a part of the inner tube 60 is accommodated in the outer tube 70, and is able to be in the extended form where the inner tube 60 is extracted from the opening portion 71 from the accommodated form. The outer tube 70 and/or the inner tube 60 includes the flexible portion 62 having lower flexural rigidity than a portion adjacent to the flexible portion 62 in the axial direction. In the accommodated form, the flexible portion 62 is located in a range where the outer tube 70 and the inner tube 60 overlap each other, and in the extended form, the flexible portion 62 is located in a range different from the range where the outer tube 70 and the inner tube 60 overlap each other. Accordingly, in the extended form, the flexible portion 62 is located outside the range where the outer tube 70 and the inner tube 60 overlap each other, so that the flexible portion 62 can be flexibly bent. For this reason, in the extended form, the flexibility of the medical device 10 between the first connecting portion 58 and the second connecting portion 59 is improved, and the passability of the medical device 10 in a tubular member such as the delivery sheath 80 or in a biological lumen is improved. In addition, in the accommodated form, the flexible portion 62 is located in the range where the outer tube 70 and the inner tube 60 overlap each other. For this reason, in the accommodated form, it is possible to make the medical device 10 difficult to bend between the first connecting portion 58 and the second connecting portion 59. The medical device 10 can be, for example, a catheter that bends easily until the catheter reaches a stenosed site and is difficult to bend at the stenosed site to exert a strong pushing force, a catheter that forms a bent or meandering lumen into a linear shape, and the like.

In addition, the form of the flexible portion 62 is not particularly limited as long as the flexible portion 62 is more flexible than a portion adjacent to the flexible portion 62 in the axial direction. For example, the flexible portion 62 may be formed as a non-through groove in the inner peripheral surface and/or in the outer peripheral surface of the inner tube 60 or the outer tube 70. In addition, as illustrated in Fig. 11(A), the flexible portion 62 may be a slit portion or a non-through groove that is formed to extend in the circumferential direction instead of having a spiral shape. In addition, as illustrated in Fig. 11(B), the flexible portion 62 may be a plurality of through-holes or a plurality of non-through holes. In addition, as illustrated in Fig. 11(C), the flexible portion 62 may be formed to be thinner than a portion adjacent to the flexible portion 62 in the axial direction. In addition, as illustrated in Fig. 11(D), the flexible portion 62 may be formed in a coil shape. In addition, as illustrated in Fig. 11(E), the flexible portion 62 may be formed by twisting or knitting a plurality of wires. In addition, the flexible portion 62 may be made of a material softer than the material of a portion adjacent to the flexible portion 62 in the axial direction. The hardness (softness) of the material can be specified by, for example, Rockwell hardness, Brinnel hardness, Vickers hardness, Shore hardness, durometer hardness, and the like.

In addition, the inner tube 60 may be connected to the second connecting portion 59 so as to be slightly movable. In addition, the outer tube 70 may be connected to the first connecting portion 58 so as to be slightly movable.

Note that this application is based on Japanese Patent Application No. 2019-179477, filed on September 30, 2019.

### Reference Signs List

10: Medical device
11: Guide wire
20: Shaft portion
21: Expansion body
30: Storage sheath
58: First connecting portion
59: Second connecting portion
60: Inner tube
61: Second engagement portion
62, 74: Flexible portion
63, 75: Slit portion
70: Outer tube
71: Opening portion
72: Open end
73: First engagement portion
74: Flexible portion

## Claims

1. A medical device (10) comprising:
an elongated shaft portion (20); and
an expansion body (21) provided at a distal portion of the shaft portion (20) to be expandable and contractable in a radial direction,
wherein the shaft portion (20) includes an outer tube (70), and an inner tube (60) that is slidable in an axial direction inside the outer tube (70),
the expansion body (21) includes a first connecting portion (58) connected to the outer tube (70), and a second connecting portion (59) connected to the inner tube (60),
the outer tube (70) includes an open end (72) at which an opening portion (71) is formed, the inner tube (60) entering and exiting from the opening portion (71),
the expansion body (21) is deformable to be in a reference form in which the expansion body (21) is widened in the radial direction in a natural state, and is deformable to be in a contracted form in which the expansion body (21) is contracted in the radial direction by the first connecting portion (58) and the second connecting portion (59) being more separated from each other in the contracted form than in the reference form, and
in the reference form, the open end (72) is located between the first connecting portion (58) and the second connecting portion (59) and when the expansion body (21) is deformed from the reference form into the contracted form, a part of the inner tube (60) is extracted from the opening portion (71),
**characterized in that**
in the reference form where the expansion body (21) is widened with no external force acting on the expansion body (21), a distance L1 from the first connecting portion (58) to the open end (72) is 30% to 80% of a distance L2 from the first connecting portion (58) to the second connecting portion (59).

2. The medical device (10) according to claim 1,
wherein the expansion body (21) is deformable to be in an expanded form, in which the expansion body (21) is expanded in the radial direction, from the reference form by the first connecting portion (58) and the second connecting portion (59) approaching each other from the reference form, and
when the expansion body (21) is deformed from the reference form into the expanded form, a part of the inner tube (60) is stored inside the outer tube (70) from the opening portion (71).

3. The medical device (10) according to claim 1 or 2,
wherein the outer tube (70) and/or the inner tube (60) includes a flexible portion (62, 74) having lower flexural rigidity than a portion adjacent to the flexible portion (62, 74) in the axial direction,
in the reference form, the flexible portion (62, 74) is located in a range in which the outer tube (70) and the inner tube (60) overlap each other, and
in the contracted form, the flexible portion (62, 74) is located in a range different from the range in which the outer tube (70) and the inner tube (60) overlap each other.

4. The medical device (10) according to any one of claims 1 to 3,
wherein the outer tube (70) includes a first engagement portion (73),
the inner tube (60) includes a second engagement portion (61), and
at least in the reference form, the first engagement portion (73) and the second engagement portion (61) are slidable in the axial direction, and come into contact with each other in a circumferential direction to limit relative rotation between the outer tube (70) and the inner tube (60).

5. The medical device (10) according to any one of claims 3 or 4,
wherein the flexible portion (62, 74) is formed of a slit portion (63, 75) or a groove having a spiral shape provided in the outer tube (70) and/or in the inner tube (60).

6. The medical device (10) according to any one of claims 3 to 5,
wherein the flexible portion (62, 74) is formed of a plurality of wires.

7. The medical device (10) according to any one of claims 3 to 5,
wherein the flexible portion (62, 74) is formed in a coil shape.

8. The medical device (10) according to any one of claims 3 to 7,
wherein the flexible portion (62, 74) is made of a material softer than a material of a portion adjacent to the flexible portion (62, 74) in the axial direction.

## Patentansprüche

1. Medizinische Vorrichtung (10), umfassend:
einen langgestreckten Schaftabschnitt (20); und
einen Ausdehnungskörper (21), der in einem distalen Abschnitt des Schaftabschnitts (20) so vorgesehen ist, dass er in einer radialen Richtung ausdehnbar und zusammenziehbar ist,
wobei der Schaftabschnitt (20) ein äußeres Rohr (70) und ein inneres Rohr (60) umfasst, das in der axialen Richtung innerhalb des äußeren Rohres (70) verschiebbar ist,
wobei der Ausdehnungskörper (21) einen ersten Verbindungsabschnitt (58), der mit dem äußeren Rohr (70) verbunden ist, und einen zweiten Verbindungsabschnitt (59) umfasst, der mit dem inneren Rohr (60) verbunden ist,
wobei das äußere Rohr (70) ein offenes Ende (72) umfasst, an dem ein Öffnungsabschnitt (71) ausgebildet ist, wobei das innere Rohr (60) in den Öffnungsabschnitt (71) eintritt und daraus austritt,
der Ausdehnungskörper (21) verformbar ist, um in einer Referenzform zu sein, in welcher der Ausdehnungskörper (21) in einem natürlichen Zustand in der radialen Richtung aufgeweitet ist, und verformbar ist, um in einer zusammengezogenen Form zu sein, in welcher der Ausdehnungskörper (21) durch den ersten Verbindungsabschnitt (58) und den zweiten Verbindungsabschnitt (59), welche in der zusammengezogenen Form weiter voneinander entfernt sind als in der Referenzform, in der radialen Richtung zusammengezogen ist, und
in der Referenzform das offene Ende (72) zwischen dem ersten Verbindungsabschnitt (58) und dem zweiten Verbindungsabschnitt (59) angeordnet ist, und wenn der Ausdehnungskörper (21) von der Referenzform in die zusammengezogene Form verformt wird, ein Teil des inneren Rohres (60) aus dem Öffnungsabschnitt (71) herausgezogen wird,
**dadurch gekennzeichnet, dass**
in der Referenzform, in welcher der Ausdehnungskörper (21) ohne Einwirkung einer äußeren Kraft auf den Ausdehnungskörper (21) aufgeweitet ist, der Abstand L1 von dem ersten Verbindungsabschnitt (58) zu dem offenen Ende (72) 30 % bis 80 % des Abstands L2 von dem ersten Verbindungsabschnitt (58) zu dem zweiten Verbindungsabschnitt (59) beträgt.

2. Medizinische Vorrichtung (10) nach Anspruch 1,
wobei der Ausdehnungskörper (21) verformbar ist, um in einer ausgedehnten Form vorzuliegen, in welcher der Ausdehnungskörper (21) in der radialen Richtung ausgedehnt ist, indem sich der erste Verbindungsabschnitt (58) und der zweite Verbindungsabschnitt (59) aus der Referenzform heraus einander annähern, und
wenn der Ausdehnungskörper (21) von der Referenzform in die ausgedehnte Form verformt wird, ein Teil des inneren Rohres (60) in dem Inneren des äußeren Rohres (70) von dem Öffnungsabschnitt (71) gelagert wird.

3. Medizinische Vorrichtung (10) nach Anspruch 1 oder 2,
wobei das äußere Rohr (70) und/oder das innere Rohr (60) einen flexiblen Abschnitt (62, 74) umfassen, der eine geringeren Biegesteifigkeit als ein Abschnitt aufweist, der in axialer Richtung zu dem flexiblen Abschnitt (62, 74) benachbart ist,
in der Referenzform der flexible Abschnitt (62, 74) in einem Bereich angeordnet ist, in welchem sich das äußere Rohr (70) und das innere Rohr (60) einander überlappen, und
in der zusammengezogenen Form der flexible Abschnitt (62, 74) in einem Bereich angeordnet ist, der sich von dem Bereich unterscheidet, in dem sich das äußere Rohr (70) und das innere Rohr (60) einander überlappen.

4. Medizinische Vorrichtung (10) nach einem der Ansprüche 1 bis 3,
wobei das äußere Rohr (70) einen ersten Eingriffsabschnitt (73) umfasst,
das innere Rohr (60) einen zweiten Eingriffsabschnitt (61) umfasst, und
mindestens in der Referenzform der erste Eingriffsabschnitt (73) und der zweite Eingriffsabschnitt (61) in der axialen Richtung verschiebbar sind und in einer Umfangsrichtung miteinander in Kontakt kommen, um die relative Drehung zwischen dem äußeren Rohr (70) und dem inneren Rohr (60) einzuschränken.

5. Medizinische Vorrichtung (10) nach einem der Ansprüche 3 oder 4,
wobei der flexible Abschnitt (62, 74) aus einem Schlitzabschnitt (63, 75) oder einer Nut, die eine Spiralform aufweist, gebildet ist, die in dem äußeren Rohr (70) und/oder in dem inneren Rohr (60) vorgesehen sind.

6. Medizinische Vorrichtung (10) nach einem der Ansprüche 3 bis 5,
wobei der flexible Abschnitt (62, 74) aus einer Vielzahl von Drähten gebildet ist.

7. Medizinische Vorrichtung (10) nach einem der Ansprüche 3 bis 5,
wobei der flexible Abschnitt (62, 74) in einer Spulenform ausgebildet ist.

8. Medizinische Vorrichtung (10) nach einem der Ansprüche 3 bis 7,
wobei der flexible Abschnitt (62, 74) aus einem Material hergestellt ist, das weicher ist als ein Material eines Abschnitts, der in der axialen Richtung benachbart zu dem flexiblen Abschnitt (62, 74) ist.

## Revendications

1. Dispositif médical (10) comprenant :
une partie tige (20) allongée ; et
un corps de déploiement (21) disposé au niveau d'une partie distale de la partie tige (20) pour être déployable et contractable dans une direction radiale,
dans lequel la partie tige (20) comporte un tube externe (70), et un tube interne (60) qui peut coulisser dans une direction axiale à l'intérieur du tube externe (70),
le corps de déploiement (21) comporte une première partie de raccordement (58) raccordée au tube externe (70), et une seconde partie de raccordement (59) raccordée au tube interne (60),
le tube externe (70) comporte une extrémité ouverte (72) au niveau de laquelle une partie d'ouverture (71) est formée, le tube interne (60) entrant et sortant de la partie d'ouverture (71),
le corps de déploiement (21) est déformable pour être dans une forme de référence dans laquelle le corps de déploiement (21) est élargi dans la direction radiale dans un état naturel, et est déformable pour être dans une forme contractée dans laquelle le corps de déploiement (21) est contracté dans la direction radiale par la première partie de raccordement (58) et la seconde partie de raccordement (59) qui sont davantage séparées l'une de l'autre dans la forme contractée que dans la forme de référence, et
dans la forme de référence, l'extrémité ouverte (72) est située entre la première partie de raccordement (58) et la seconde partie de raccordement (59) et lorsque le corps de déploiement (21) est déformé par rapport à la forme de référence dans la forme contractée, une partie du tube interne (60) est extraite de la partie d'ouverture (71),
**caractérisé en ce que**
dans la forme de référence dans laquelle le corps de déploiement (21) est élargi sans force externe agissant sur le corps de déploiement (21),
une distance L1 de la première partie de raccordement (58) à l'extrémité ouverte (72) est de 30 % à 80 % d'une distance L2 de la première partie de raccordement (58) à la seconde partie de raccordement (59).

2. Dispositif médical (10) selon la revendication 1,
dans lequel le corps de déploiement (21) est déformable pour être sous une forme déployée, dans lequel le corps de déploiement (21) est déployé dans la direction radiale, à partir de la forme de référence par la première partie de raccordement (58) et la seconde partie de raccordement (59) qui s'approchent l'une de l'autre à partir de la forme de référence, et
lorsque le corps de déploiement (21) est déformé par rapport à la forme de référence dans la forme déployée, une partie du tube interne (60) est stockée à l'intérieur du tube externe (70) à partir de la partie d'ouverture (71).

3. Dispositif médical (10) selon la revendication 1 ou 2, dans lequel le tube externe (70) et/ou le tube interne (60) comportent une partie flexible (62, 74) présentant une rigidité de flexion inférieure à celle d'une partie adjacente à la partie flexible (62, 74) dans la direction axiale,
dans la forme de référence, la partie flexible (62, 74) est située dans une plage dans laquelle le tube externe (70) et le tube interne (60) se chevauchent, et
dans la forme contractée, la partie flexible (62, 74) est située dans une plage différente de la plage dans laquelle le tube externe (70) et le tube interne (60) se chevauchent.

4. Dispositif médical (10) selon l'une quelconque des revendications 1 à 3,
dans lequel le tube externe (70) comporte une première partie de mise en prise (73),
le tube interne (60) comporte une seconde partie de mise en prise (61), et
au moins dans la forme de référence, la première partie de mise en prise (73) et la seconde partie de mise en prise (61) sont coulissantes dans la direction axiale, et entrent en contact l'une avec l'autre dans une direction circonférentielle pour limiter une rotation relative entre le tube externe (70) et le tube interne (60).

5. Dispositif médical (10) selon l'une quelconque des revendications 3 ou 4,
dans lequel la partie flexible (62, 74) est formée d'une partie fendue (63, 75) ou d'une rainure présentant une forme de spirale disposée dans le tube externe (70) et/ou dans le tube interne (60).

6. Dispositif médical (10) selon l'une quelconque des revendications 3 à 5,
dans lequel la partie flexible (62, 74) est formée d'une pluralité de fils.

7. Dispositif médical (10) selon l'une quelconque des revendications 3 à 5,
dans lequel la partie flexible (62, 74) est formée sous une forme de bobine.

8. Dispositif médical (10) selon l'une quelconque des revendications 3 à 7,
dans lequel la partie flexible (62, 74) est composée d'un matériau plus souple qu'un matériau d'une partie adjacente à la partie flexible (62, 74) dans la direction axiale.
